# EUROPÄISCHE PATENTANMELDUNG

(11) **EP 3 354 718 A1**
(43) Veröffentlichungstag der Anmeldung: **01.08.2018**
(21) Anmeldenummer: 17153818.4
(22) Anmeldetag: 30.01.2017
(51) Int. Cl.: C12M 1/107, C12M 1/00, C12M 1/33

(54) **VERFAHREN UND VORRICHTUNG ZUM ERZEUGEN VON BIOGAS**

(71) Anmelder: HERBST Umwelttechnik GmbH, 14167 Berlin (DE)
(72) Erfinder: SPAHR, Marcel, 12205 Berlin (DE); FECHTER, Dr. Leonhard, 14195 Berlin (DE)
(74) Vertreter: Pfenning, Meinig & Partner mbB

(57) **Zusammenfassung**

Die Erfindung betrifft ein Verfahren und eine Biogasanlage zum Erzeugen von Biogas, vorzugsweise aus Reisstroh, wobei ein Substrat in zwei Reaktoren (1, 2) zirkulierend vergärt wird, sodass eine Methanproduktion aus cellulose- und/oder lignocellulosehaltigem Substrat verbessert werden kann.

## Beschreibung

Die Erfindung betrifft ein Verfahren und eine Vorrichtung zum Erzeugen von Biogas.

Biogas wird in Biogasanlagen erzeugt, die ihren Einsatz beispielsweise in der Landwirtschaft finden und mit meist tierischen Exkrementen und Energiepflanzen als Substrat gespeist werden. Bei der Vergärung des organischen Materials entsteht das Biogas als Stoffwechselprodukt von Bakterien und Mikroorganismen. Biogas ist ein Gasgemisch aus Methan (CH₄) und Kohlendioxid (CO₂), mit bis zu 2% Schwefelwasserstoff (H₂S) und Spurengasen wie Ammoniak (NH₃), Wasserstoff (H₂), Stickstoff (N₂) und Kohlenmonoxid (CO).

Biogas kann aus nahezu allen stark wasserhaltigen organischen Stoffen mit den Grundbestandteilen Fett, Eiweiß und Kohlenhydraten erzeugt werden. Stroh und Holz, die vor allem Cellulose und Lignocellulose enthalten, sind nur schwer abbaubar und werden daher wenig eingesetzt oder einer Vorbehandlung unterzogen. Insbesondere Reisstroh ist aufgrund hoher Lignin- und Silikatanteile im Vergleich zu leicht vergärbaren Rohstoffen wie Maissilage schwer aufzuschließen und erzielt in herkömmlichen Biogasanlagen somit geringere Biogaserträge.

Die EP 2 927 308 A1 betrifft ein Verfahren und eine Biogasanlage zur Erzeugung von Biogas aus Stroh. Dabei sind Mittel zur Vorbehandlung des Strohs vorgesehen, um einen mechanischen Aufschluss desselben zu bewirken, bevor das Stroh in einen Fermenter eingebracht wird, in dem eine anaerobe bakterielle Vergärung stattfindet. Die Vorbehandlung des Strohs durch eine Vermahlung in einer Hammermühle, wie sie beispielsweise die EP 2 927 308 A1 vorschlägt, ist kostenintensiv und führt zu einem Verkleben der Gärmasse, sodass eine Perkolation und damit eine Biogasbildung gehemmt werden.

Der Erfindung liegt daher die Aufgabe zugrunde, ein verbessertes Verfahren zur Biogasherstellung vorzuschlagen. Insbesondere kann der Erfindung die Aufgabe zugrunde liegen, ein Verfahren hinsichtlich Wirtschaftlichkeit zu verbessern. Des Weiteren kann der Erfindung zudem die Aufgabe zugrunde liegen, ein Verfahren vorzuschlagen, dass eine Biogasherstellung aus Stroh, verbessert und/oder eine Biogasherstellung aus Reisstroh ermöglicht. Ferner kann die Erfindung die Aufgabe haben, eine Biogasanlage zur Durchführung des Verfahrens vorzuschlagen.

Die Aufgabe wird gelöst durch ein Verfahren gemäß Anspruch 1 sowie durch eine Biogasanlage mit den Merkmalen des nebengeordneten Anspruches. Vorteilhafte Weiterbildungen ergeben sich mit den Merkmalen der Unteransprüche und der Ausführungsbeispiele.

Das vorgeschlagene Verfahren zum Erzeugen von Biogas umfasst dabei folgend beschriebene Schritte. Die beschriebenen Schritte müssen nicht in der aufgeführten Reihenfolge ausgeführt werden.

Ein cellulosehaltiges und/oder lignocellulosehaltiges Substrat, wie zum Beispiel Stroh oder Reisstroh, wird in einem ersten Reaktor mit mesophilen Bakterien während einer ersten Verweilzeit zumindest teilweise vergoren. Die Temperatur im Reaktor beträgt dabei mindestens 20°C, vorzugsweise mindestens 25°C, besonders bevorzugt mindestens 30°C und/oder höchstens 50°C, bevorzugt höchstens 43°C, besonders bevorzugt höchstens 46°C. Die Temperatur kann durch ein Erwärmen des Inhalts des ersten Reaktors eingestellt werden und/oder kann sich zumindest teilweise durch exotherme Reaktionen der bakteriellen Stoffwechselprozesse bei der Methanherstellung einstellen.

Die mesophilen Bakterien eignen sich, um aus Essigsäure Methan herzustellen. Anzumerken ist, dass "Bakterien" hier in einem weiteren Sinne gemeint sind. Wissenschaftlich ist es umstritten, ob Archaeen und Bakterien verschiedene Taxa sind. Hier sind unter Bakterien jedoch insbesondere auch Archaeen zu verstehen. Dabei können die Archaeen vorzugsweise der Ordnung der Methanobacteriales, Methanococcales, Methanomicrobiales, Methanocellales, Methanosarcinales, Methanoscarcina, Methanococcus, Methanobacterium Methanobrevibacter, Methanothermobacter und/oder der Methanopyrale sein. Die Bakterien können sowohl acetatspaltende Methanogene als auch Wasserstoff-oxidierende Methanogene umfassen. Acetatspaltende Methanogene bilden Methan beispielsweise aus Methylgruppenhaltigen Verbindungen, beispielsweise Essigsäure, indem sie die Methylgruppe abspalten und zu Methan reduzieren. Wasserstoff-oxidierende Methanogene reduzieren indes Kohlenstoffdioxid mit Wasserstoff zu Methan und Wasser sowie durch Umsetzung von Ameisensäure (HCOOH). Der erste Reaktor ist vorzugsweise luftdicht abgeschlossen, sodass eine anaerobe Vergärung sichergestellt werden kann.

Die erste Verweilzeit ist eine Zeit, die das Substrat in dem ersten Reaktor verbleibt. Diese Zeit kann das Substrat am Stück in dem ersten Reaktor verbleiben, oder es kann mit einer ersten Verweilzeit auch eine additive Zeit gemeint sein, die diejenige Zeit beschreibt, die eine Teilmenge des Substrats im ersten Reaktor zwischen einem Zeitpunkt eines erstmaligen Einbringens der Teilmenge des Substrats in die Biogasanlage und einem Zeitpunkt eines Auslassens der Teilmenge des Substrats aus der Biogasanlage verbleibt. Dabei beträgt die bevorzugte Verweilzeit mindestens 20 Tage, vorzugsweise mindestens 25 Tage und/oder höchstens 40 Tage, vorzugsweise höchstens 35 Tage. Bei dem cellulosehaltigen und /oder lignocellulosehaltigen Substrat handelt es sich typischerweise um Stroh, vorzugsweise um Reisstroh. Es können aber auch Mischverhältnisse von cellulosehaltigen und/oder lignocellulosehaltigen Substraten und leicht vergärbaren Substraten wie beispielsweise Gülle, Rotte oder Speisereste verwendet werden. Dabei kann ein Masseverhältnis von cellulosehaltigen und/oder lignocellulosehaltigen Substraten m_{C/LC} zu einem Masseverhältnis von leicht vergärbaren Substraten mₗᵥ mindestens m_{C/LC}/mₗᵥ=50/1, vorzugsweise mindestens m_{C/LC}/mₗᵥ=40/1, besonders vorzugsweise m_{C/LC/}mₗᵥ=20/1 in Gewichtsteilen sein.

Ein weiterer Verfahrensschritt ist ein Weiterleiten eines Teils des zumindest teilweise vergorenen Substrats aus dem ersten Reaktor in einen zweiten, beheizbaren Reaktor mit hyperthermophilen Bakterien, wobei sich die hyperthermophilen Bakterien dazu eignen, das zumindest teilweise vergorenen Substrat aufzuschließen. Hyperthermophile Bakterien haben Wachstumsoptima bei Temperaturen von mehr als 60°C. Bei den hyperthermophilen Bakterien handelt es sich dabei vorzugsweise um Bakterien aus der Gruppe der Chlostridien, vorzugsweise Chlostridium aceticum, Chlostridium thermocellum und/oder Chlostridium stecorarium. Diese können in Symbiose mit anderen Spezies zumindest teilweise Lignocellulose aufschließen und zu niederen organischen Säuren und kurzkettigen Alkoholen verstoffwechseln. Diese Zwischenprodukte können sein: Essigsäure, Ameisensäure, Propionsäure, Buttersäure, Ethanol, Butanol und/oder Butandiol. Diese Zwischenprodukte können im Anschluss von acetogenen Bakterien zu Wasserstoff, Kohlendioxid und vornehmlich Essigsäure weiter abgebaut werden. Der zweite Reaktor kann dabei luftdicht abgedichtet sein, sodass eine anaerobe Vergärung unter Ausschluss von Sauerstoff stattfinden kann.

Das zumindest teilweise vergorene Substrat wird bei einer Temperatur im Bereich von mindestens 55°C, vorzugsweise mindestens 60°C, besonders vorzugsweise mindestens 65°C und/oder höchstens 80°C, vorzugsweise höchstens 75°C, besonders vorzugsweise höchstens 70°C für eine zweite Verweilzeit inkubiert. Dabei wird zumindest teilweise Essigsäure gebildet. Typischerweise beträgt die zweite Verweilzeit des Substrats im zweiten Reaktor mindestens einen Tag und/oder höchstens 5 Tage, vorzugsweise mindestens zwei Tage und/oder höchstens drei Tage. Auch die zweite Verweilzeit kann - wie auch die erste Verweilzeit - absolut oder additiv sein. Das Substrat kann also für eine zweite Verweilzeit am Stück im zweiten Reaktor verbleiben oder eine Summe einzelner Zeitabschnitte, die das Substrat im zweiten Reaktor verbringt, entspricht der zweiten Verweilzeit.

Aus dem zweiten Reaktor wird das Substrat mit der gebildeten Essigsäure in den ersten Reaktor rückgeführt. Im Substrat enthaltende Lignocellulose oder Cellulose wurde nach dem Inkubieren im zweiten Reaktor zumindest teilweise zu beispielsweise Essigsäure aufgeschlossen. Lignocellulose, die in der zweiten Inkubationszeit nicht zu Essigsäure umgesetzt worden ist, wurde von Mikroorganismen zumindest teilweise zu niederen Strukturen aufgeschlossen. Der rückgeführte Schlamm mit gebildeter Essigsäure wird im ersten Reaktor inkubiert, und es wird insbesondere die Essigsäure, wie oben beschrieben, von den mesophilen Bakterien zu Methan umgesetzt.

Methanhaltiges Biogas wird in einem weiteren Schritt aus dem ersten Reaktor isoliert. Das Biogas enthält dabei typischerweise 40% bis 60% Methan (CH₄), 40% bis 60% Kohlenstoffdioxid (CO₂), 100 bis 5000ppm Schwefelwasserstoff (H₂S) und Spurengase.

Ein Substrataufnahmevolumenverhältnis, also ein Verhältnis zwischen dem Volumen, das ein Reaktor an Substrat aufnehmen kann, zwischen dem zweiten und dem ersten Reaktor beträgt typischerweise etwa V₂/V₁ = 1/20, vorzugsweise etwa V₂/V₁ = 1/6, besonders vorzugsweise etwa V₂/V₁ = 1/15. Der erste Reaktor kann also beispielsweise 1000 m³ Substrat aufnehmen, während der zweite Reaktor 50 m³ Substrat, besonders vorzugsweise 100 m³ Substrat aufnehmen kann. Ein vorteilhaftes Verhältnis von V2 zu V1 kann mit 1/6 bis max. 1/20 angegeben werden.

Eine Raumbelastung beschreibt eine Menge an organischer Trockensubstanz (oTS) gemessen am Reaktorvolumen über eine Zeit in Tagen. Die Raumbelastung im ersten Reaktor ist typischerweise mind. 2,5 (kg oTS)/(m³ d), vorzugsweise mind. 3 (kg oTS)/(m³ d). besonders vorzugsweise 3,5 (kg oTS)/(m³ d) und/oder maximal 5,0 (kg oTS)/(m³ d), vorzugsweise max. 4,5 (kg oTS)/(m³ d).

Die Raumbelastung des zweiten Reaktors ist in einigen Ausführungsbeispielen 5 bis 20 mal so hoch, vorzugsweise 7 bis 13 mal so hoch, besonders bevorzugt 9 bis 11 mal so hoch, wie die Raumbelastung im ersten Reaktor.

Eine Feststoffzufuhr in Form von Stroh, insbesondere Reisstroh, in den ersten Reaktor beträgt vorzugsweise mindestens 10 Gew% im Zulaufstrom, besonders bevorzugt zwischen 20 Gew% und 40 Gew%, maximal bis zu 70 Gew%. Hinzu kommen vorzugsweise 30 bis 90 Gew% Gülle, beispielsweise Rindergülle, besonders bevorzugt 60Gew%. Alternativ zu Güllen und/oder Wasser kann Rezirkulat zur Flüssigkeitsbereitstellung hinzugegeben werden, vorzugsweise 5 bis 80Gew%, besonders bevorzugt 10 bis 70Gew%.
In ihrer Gesamtheit können die vorgenannten Feststoffe vorzugsweise 100 % ergeben.

Das Substrat kann im ersten und/oder im zweiten Reaktor mittels einer Pump- und/oder Rühreinrichtung durchmischt, vorzugsweise kontinuierlich durchmischt werden. Dies kann vorteilhaft sein, um Ablagerungen und ein Verkleben des Substrats zu verhindern, sodass eine möglichst gleichmäßige Temperatur und Nährstoffverteilung generiert werden kann.

Ferner kann das Verfahren zumindest teilweise zirkulierend durchgeführt werden, also die Schritte "Weiterleiten eines Teils des zumindest teilweise vergorenen Substrats aus dem ersten Reaktor in einen zweiten, beheizbaren Reaktor mit hyperthermophilen Bakterien, wobei sich die hyperthermophilen Bakterien dazu eignen, das zumindest teilweise vergorene Substrat aufzuschließen" und "Rückführen des Substrats mit Essigsäure aus dem zweiten Reaktor in den ersten Reaktor" wiederholt werden. So kann eine Zirkulation von Substrat generiert werden und das Substrat kann mit jedem erneuten Durchlaufen eines Reaktors weiter aufgeschlossen werden. Die Reaktionen in den Reaktoren können einander beeinflussen, da beispielsweise das Reaktionsprodukt im zweiten Reaktor (Essigsäure) im ersten Reaktor ein Reaktant ist. Für eine Methanproduktion ist also unmittelbar eine Methanproduktion im ersten Reaktor verantwortlich, mittelbar ist jedoch auch ein Cellulose- oder Lignolcelluloseaufschluss im zweiten Reaktor nötig. Ein vorteilhafter, weiterer Aufschluss, insbesondere von schwer vergärbarem Substrat wie beispielsweise Lignocellulose, kann also eine erhöhte Methanproduktion generieren, als ein einmaliger Durchlauf von Substrat durch den ersten und/oder zweiten Reaktor.

Im ersten Reaktor verbleibende Gärreste, die nach einer Vergärung im ersten Reaktor im Wesentlichen nicht weiter durch die Bakterien aufgeschlossen werden, können aus dem ersten Reaktor ausgeleitet und drainiert werden.

Über eine Messeinrichtung, die misst, wie hoch ein Biogasertrag zu einem momentanen Zeitpunkt ist, kann beispielsweise ermittelt werden, wann ein Biogasertrag abfällt. Wenn nicht mehr genug oder nur noch wenig Biogas produziert wird, können Substratreste, die im Reaktor verbleiben weitestgehend nicht mehr weiter aufgeschlossen werden. Diese Substratreste sind demnach Gärreste. Ein Zeitpunkt, zu dem die Gärreste ausgeleitet werden sollten, kann auch festgelegt sein und nicht durch einen Biogasertrag bestimmt werden. Beispielsweise können regelmäßig nach der ersten Verweilzeit Gärreste ausgeleitet werden. Über eine Ableitung kann dabei eine entsprechend separierte Prozessflüssigkeit dem ersten Reaktor zumindest teilweise wieder zugeführt werden. Gärreste sind dabei flüssige oder feste Rückstände, die bei der Vergärung von Substrat zurückbleiben. Beim Drainieren können Flüssigkeiten von den festen Gärresten derart separiert werden, dass ein fester Gärrest und eine Prozessflüssigkeit entstehen. Feste Gärreste können aus der Biogasanlage ausgeschleust und die Prozessflüssigkeit kann einer Mischvorrichtung oder direkt dem ersten Reaktor zugeführt werden. Die Ableitung kann somit in eine Entwässerungsvorrichtung und von dort entweder in den ersten Reaktor oder in die Mischvorrichtung führen. In der Mischvorrichtung kann Substrat, das dem ersten Reaktor zugeführt werden soll mit der Prozessflüssigkeit gemischt werden, um eine homogenere Substratkonsistenz zu erreichen und/oder ein Trockensubstanzgehalt einstellen zu können. Die Trockensubstanz ist dabei jener Anteil einer Substanz, der nach vollständigem Entzug von Wasser übrigbleibt. Aus der Mischvorrichtung kann ein Substrat- und Prozessflüssigkeitsgemisch in den ersten Reaktor geleitet werden.

Aus dem zweiten Reaktor können etwaige Störstoffe ausgeschleust werden. Kieselsäure kann beispielsweise beim Abbau von Biomasse als unerwünschtes Produkt anfallen. Über Zirkulationseinrichtungen können diese in den ersten Reaktor gelangen und dort methanbildungshemmend wirken. Zum Herabsetzen einer Konzentration von methanbildungshemmender Kieselsäure und/oder anderer Bakterien schädlicher Substanzen kann diese deshalb vorzugsweise schon im zweiten Reaktor ausgeschleust werden. Dies kann somit eine Methanbildung begünstigen und sich positiv auf einen Wirkungsgrad der Biogasanlage auswirken.

Ferner kann, wie bereits erwähnt, das cellulosehaltige Substrat Lignocellulose enthalten. Bevorzugt kann dabei strohartige Biomasse, beispielsweise Stroh, insbesondere Weizenstroh, Heu, und /oder Reisstroh und/oder dessen Silagen als Substrat verwendet werden. Insbesondere Reisstroh eignet sich, da hier ein hoher Energiegehalt durch einen hohen Anteil an Lignocellulose vorhanden ist. Durch einen Aufschluss der Lignocellulose im zweiten Reaktor kann diese Energie im ersten Reaktor in Form von Biogas nutzbar gemacht werden. Durch Silagen sind dabei durch Milchsäurebakterien konservierte Substrate, wobei Milchsäurebakterien den im Substrat enthaltenen Zucker in Säuren umgewandelt haben. Zudem kann die Biogasanlage auch mit Gülle oder gülleartigen Reststoffen beschickt werden, vorzugsweise mit Hühner-, Schweine- oder Rindergülle.

Typischerweise liegt ein pH-Wert des Substrats im ersten Reaktor im pHneutralen bis leicht basischen Bereich, sodass mesophile Bakterien unter möglichst günstigen Lebensbedingungen leben können, um eine Methanbildung zu begünstigen. Günstige Lebensbedingungen liegen typischerweise zwischen pH 6,6 und 8,3.

Im zweiten Reaktor beträgt ein pH-Wert typischerweise mindestens 3,5, vorzugsweise mindestens 4,5 und/oder höchstens 7,5; vorzugsweise höchstens 6,5 beträgt. Ein derartiger pH-Wert kann eine Bildung von Essigsäure im zweiten Reaktor durch die hyperthermophilen Bakterien begünstigen. Im ersten Reaktor kann wiederum aus der Essigsäure Methan gebildet werden, sodass eine höhere Essigsäureproduktion mittelbar eine Methanbildung begünstigt.

Typischerweise können Substratfasern vor dem Einbringen des Substrats in den ersten Reaktor zerkleinert werden. Die Substratfasern können beispielsweise mit einer Schneidemühle mechanisch vorbehandelt werden. Reisstroh wird dabei typischerweise auf eine Faserlänge von 40 mm zugeschnitten werden, wobei natürlich sowohl für Reisstroh als auch für andere Substratfasern andere Faserlängen, vorzugsweise kürzer als 60 mm, denkbar sind. Eine derartige Zerkleinerung kann den Vorteil haben, dass sich Substratfasern nicht um Rührvorrichtungen oder Rotoren von Pumpen wickeln können und damit deren Funktion beeinträchtigen können.

Die Erfindung betrifft ferner eine Biogasanlage zur Erzeugung von Biogas, enthaltend einen ersten Reaktor, einen zweiten Reaktor und eine Zirkulationseinrichtung. Der erste Reaktor enthält dabei mesophile Bakterien, die sich zur Methanherstellung aus Essigsäure, Wasserstoff und Kohlendioxid eignen. Der zweite Reaktor ist beheizbar und enthält hypothermophile Bakterien, die sich zum zumindest teilweise anaeroben Vergären von einem cellulosehaltigen, vorzugsweise lignocellulosehaltigen, Substrat in Essigsäure eignen. Die Zirkulationseinrichtung zum Erzeugen eines Biomassekreislaufs zwischen dem ersten Reaktor und dem zweiten Reaktor umfasst eine Fördereinrichtung und zumindest eine den ersten Reaktor und den zweiten Reaktor verbindende Verbindungsleitung. Die Fördereinrichtung ist eingerichtet zum Fördern von Substrat durch die Verbindungsleitung sowohl von dem ersten Reaktor in den zweiten Reaktor als auch von dem zweiten Reaktor in den ersten Reaktor. Die Zirkulationseinrichtung kann dabei eine Pumpvorrichtung aufweisen, die Substrat von einem Reaktor in den anderen pumpt. Dabei kann die Pumpvorrichtung beispielsweise eine Schneckenexzenterpumpe aufweisen.

Die Verbindungsleitung zum Fördern von Substrat von dem ersten in den zweiten Reaktor kann eine Belüftungseinrichtung umfassen. Diese kann vorteilhaft sein, damit anaerobe Bakterienstämme des ersten Reaktors zumindest teilweise abgetötet bzw. inaktiviert werden. In einigen Ausführungsbeispielen ist in der Verbindungsleitung jeweils vor und nach der Belüftungseinrichtung ein Ventil, vorzugsweise ein Ein-Wege-Ventil, und eine Pumpe vorgesehen. So kann beispielsweise bei geschlossenem Ventil hinter der Belüftungseinrichtung mit dieser Pumpe Substrat aus dem ersten Reaktor durch ein vor der Belüftungsvorrichtung geöffnetes Ventil in die Belüftungsvorrichtung gepumpt und dort belüftet werden. Die Belüftungsvorrichtung kann optional gegenüber dem Austritt der Verbindungsleitung aus dem ersten Reaktor höher gelegen sein, sodass die Pumpe das Substrat derart nach oben (also in einer der Gewichtskraft entgegen gerichteten Richtung) pumpt, dass das Substrat aufgrund der hydrostatischen Druckgleichung mit einem Druck beaufschlagt wird. Dies kann beispielsweise durch ein Pumpen des Substrats in eine Belüftungseinrichtung in Form einer vertikalen Säule realisiert werden. Anschließend dem Belüften oder nachdem die Belüftungsvorrichtung gefüllt ist, wird das Ventil vor der Belüftungsvorrichtung geschlossen. Nach dem Belüften wird das Ventil hinter der Belüftungsvorrichtung geöffnet. In denjenigen Ausführungsformen, in denen das Substrat mit einem Druck beaufschlagt ist, fließt das Substrat durch die Druckdifferenz und das Bestreben des Substrats nach einem Druckausgleich bei dem geöffneten zweiten Ventil in den zweiten Reaktor. Andere Ausführungsformen können auch vorsehen, das Substrat mittels einer weiteren Pumpe aus der Belüftungsvorrichtung in den zweiten Reaktor zu pumpen.

Der zweite Reaktor kann in einigen Ausführungsformen eine weitere Zuleitung umfassen. Über diese Zuleitung können beispielsweise leicht vergärbare Substanzen direkt in den zweiten Reaktor geleitet werden, um den dort ablaufenden Gärprozess zu optimieren und/oder ein Verhältnis von schwervergärbaren zu leicht vergärbaren Substanzen zu regulieren. Eine pH-Wert-Regulierung in den sauren Bereich kann somit begünstigt werden. Eine Zugabe von Chemikalien kann somit nicht notwendig sein.

Ferner kann die Biogasanlage eine Entwässerungsvorrichtung zum Separieren einer Prozessflüssigkeit bei einem Entwässern von Gärresten aufweisen. Dabei kann der erste Reaktor zum Abführen der Gärreste mit der Entwässerungsvorrichtung über eine Ableitung verbunden sein. Die Ableitung kann beispielsweise als Rohrleitung oder als Schlauch ausgebildet sein. Auch ein Abpumpen der Gärreste in einen zusätzlichen Behälter ist denkbar.

Die Gärreste sind diejenigen Substratreste, die nach einer Vergärung im ersten Reaktor und/oder zweiten Reaktor im Wesentlichen nicht weiter durch die mesophilen und/oder hypothermophilen Bakterien aufgeschlossen werden.

Die Biogasanlage kann eine dem ersten Reaktor vorgeschaltete und mit dem ersten Reaktor durch eine Zuleitung verbundene Mischvorrichtung aufweisen. So kann Substrat mit einer Prozessflüssigkeit derart gemischt werden, dass ein Trockensubstanzgehalt des durch die Zuleitung dem ersten Reaktor zuführbaren Substrats einstellbar ist. Ein Trockensubstanzgehalt beträgt dabei typischerweise mindestens 8 %, vorzugsweise mindestens 12% und/oder höchstens 30%, vorzugsweise höchstens 20%.

Ferner kann die Biogasanlage eine Rückführungsvorrichtung zum Rückführen der Prozessflüssigkeit in den ersten Reaktor umfassen. Die Prozessflüssigkeit kann in die Mischvorrichtung und/oder in den ersten Reaktor geleitet werden und kann somit mit Substrat vermischt werden, das vergoren werden soll. Dabei kann die Prozessflüssigkeit insofern sinnvoll sein, als dass sie schwer vergärbare Substrate verflüssigt und homogenisiert, sodass die Bakterien besser an das Substrat gelangen und eine Vergärung begünstigt wird.

Die Verbindungsleitung zwischen dem ersten Reaktor und dem zweiten Reaktor weist in manchen Ausführungsformen eine Zwei-Wege-Ventil-Anordnung und/oder Belüftungsvorrichtung auf. Zusätzlich oder alternativ kann die Zirkulationseinrichtung auch zwei Verbindungsleitungen zwischen dem ersten Reaktor und dem zweiten Reaktor umfassen. So kann eine Zirkulation von Substrat zwischen dem ersten und dem zweiten Reaktor generiert werden. Das Substrat, das von den mesophilen Bakterien nicht oder nur schwer zu Methan verstoffwechselt werden kann, wird im zweiten Reaktor weiter aufgeschlossen. Dabei wird insbesondere lignocellulosehaltiges Substrat im zweiten Reaktor weiter aufgeschlossen. Nach einem Aufschluss des vorzugsweise lignocellulosehaltigen Substrats kann es durch die Verbindungsleitungen zurück in den ersten Reaktor geleitet werden, um dort zumindest teilweise zu Methan zu vergären. Dieser Zyklus kann beliebig oft wiederholt werden.

Zum Fördern des Substrats kann die Zirkulationseinrichtung eine Pumpe, beispielsweise eine Schneckenexzenterpumpe umfassen. Natürlich sind auch jegliche andere Pumpensysteme denkbar, die geeignet sind zumeist zähflüssige Biomasse zu fördern, beispielsweise Drehkolbenpumpen.

Die Biogasanlage kann typischerweise zumindest eine Rühreinrichtung und/oder Pumpeinrichtung zur Durchmischung des Substrats im ersten Reaktor und/oder zweiten Reaktor aufweisen. Die Rühreinrichtung können Rührwerke sein, beispielsweise Zentralrührwerke, Tauchmotorrührwerke und/oder Paddelrührwerke. Natürlich können auch andere Rührwerke genutzt werden. Dabei können vorteilhaft die Fließeigenschaften des Substrats beziehungsweise der Biomasse verbessert werden, eine im Wesentlichen konstante Zusammensetzung der Biomasse gewährleistet werden und damit eine Vergärungsprozess verbessert werden.

Der erste Reaktor kann derart beheizbar ist, dass eine Temperatur von mindestens 30°C, vorzugsweise mindestens 35°C, besonders bevorzugt mindestens 40°C einstellbar ist. Der zweite Reaktor kann derart beheizbar sein, dass eine Temperatur von mindestens 55°C, vorzugsweise mindestens 60°C, besonders vorzugsweise mindestens 65°C einstellbar ist.

In einer weiteren Ausführung kann die Biogasanlage eine Vorbehandlungseinrichtung zum Zerkleinern von Substratfasern umfassen. Substratfasern können darin auf eine einstellbare Faserlänge, vorzugsweise max. 40 mm, beschnitten werden, sodass eine im Wesentlichen homogene Durchmischung des Substrats durch Pump- oder Rührwerke erreicht werden kann und ein Umwickeln von Rührschaufeln durch Substratfasern oder ein Verstopfen von Rohrleitungen oder Pumpen durch Verklebungen oder verknoteten Substratfasern entgegengewirkt werden kann.

Der zweite Reaktor weist vorzugsweise Bakterien ausgewählt aus der Gruppe Clostridium spp. auf, vorzugsweise Clostridium aceticum, Clostridium thermocellum und/oder Clostridium stecorarium und/oder Thermotogaceae enthalten.

Das Animpfen des hyperthermophilen Reaktors erfolgt in einigen Ausführungsformen vor einer Erstbeschickung mit Substraten. Eine Animpfkultur kann beispielsweise aus bestehenden hyperthermophilen Reaktoren gemäß dem beschriebenen Patentanspruch entnommen werden (sog. Impfschlamm). Ferner ist eine Anzucht von hyperthermophilen Mikroorganismen mittels der Zugabe von tierischen Reststoffen und/oder Biomassen aus an-/aeroben Abbauprozessen (Faulung, Kompostierung, Atmungsprozessen) im zweiten Reaktor möglich.

Die beschriebene Biogasanlage kann zur Durchführung des beschriebenen Verfahrens verwendet werden. Die beschriebenen Merkmale bezüglich des Verfahrens können entsprechend für die Biogasanlage und die bezüglich der Biogasanlage beschriebenen Merkmale können ferner für das Verfahren gelten.

In bestehenden biologischen Behandlungssystemen kann nicht ausgeschlossen werden, dass leicht vergärbare Substanzen (Hexosen, Stärke etc.) durch Atmungsprozesse (aerob) zu Kohlendioxid verstoffwechselt werden. Eine Bildung von energiereichem Methan könnte über diese Stoffe ausbleiben.

Dies wird im beschriebenen Verfahren vermieden, da Frischmassen zunächst anaerob anverdaut/vergoren werden. Aerobe Atmungsprozesse finden nicht statt. Lediglich die schwer abbaubaren Substanzen (vorzugsweise Cellulosen, Hemicellosen, Lignocellosen und/oder Wachse) stehen den spezifischen Mikroorganismen zur Bildung von org. Säuren und Alkoholen zur Verfügung.
Die Wärmemengezufuhr ist gegenüber bestehenden Systemen gleichzusetzen. Eine verstärkte Energiezufuhr findet nicht statt.

Ausführungsbeispiele der vorliegenden Erfindung werden nachfolgend unter Bezugnahme auf die begleitenden Abbildungen näher erläutert. Es zeigen:
- Fig. 1a:: eine schematische Darstellung einer Biogasanlage,
- Fig. 1b:: schematische Darstellung der Biogasanlage mit einer Belüftungsvorrichtung,
- Fig. 2a:: eine schematische Darstellung einer Biogasanlage mit einer 2-Wege-Ventil-Anordnung,
- Fig. 2b:: eine schematische Darstellung einer Biogasanlage mit einer 2-Wege-Ventil-Anordnung und einer Belüftungsvorrichtung,
- Fig. 3:: eine schematische Darstellung einer Biogasanlage mit einer Entwässerungsvorrichtung,
- Fig. 4:: eine schematische Darstellung einer Biogasanlage mit einer Mischvorrichtung, einer Zerkleinerungsvorrichtung und einer Entwässerungsvorrichtung, und
- Fig. 5:: eine schematische Darstellung eines Verfahrens zum Erzeugen von Biogas.

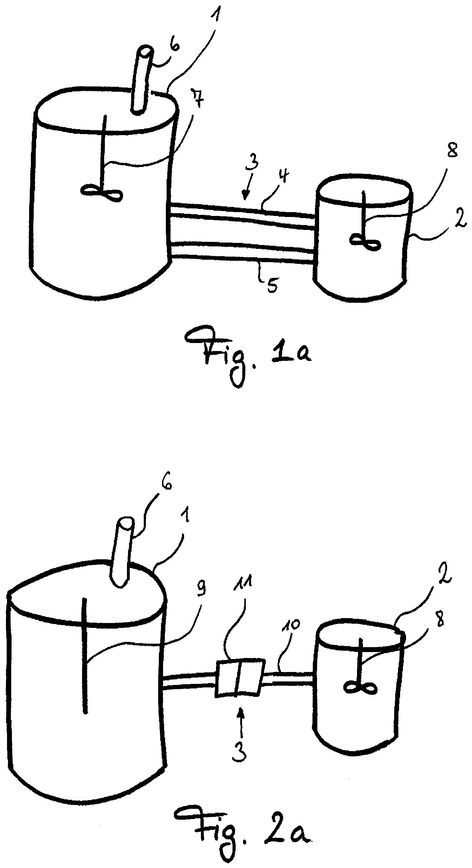

Figur 1a zeigt eine Biogasanlage mit einem ersten Reaktor 1 und einem zweiten Reaktor 2. Ein Inhalt des ersten Reaktors 1 enthält mesophile Bakterien und ein cellulose- und lignocellulosehaltiges Substrat, wobei das cellulose- und lignocellulosehaltige Substrat Reisstroh, Reisstrohsilage und Hühnergülle umfasst. Der erste Reaktor 1 ist im gezeigten Beispiel beheizbar und der Inhalt des ersten Reaktors 1 wurde auf eine Temperatur von 33°C erwärmt. Es ist jedoch auch denkbar, dass der erste Reaktor 1 nicht beheizbar ist und sich eine Temperatur zwischen 20°C und 40°C durch einen exothermen Vergärungsprozess durch die enthaltenden mesophilen Bakterien einstellt. Die mesophilen Bakterien - im vorliegenden Beispiel handelt es sich um Archaeen, und zwar Methanobacteriales, Methanococcales, Methanomicrobiales und Methanocellales - eignen sich, um aus Essigsäure Methan herzustellen. Das im ersten Reaktor 1 enthaltene Substrat verweilt beispielhaft 30 Tage im ersten Reaktor 1 und vergären zumindest teilweise, sodass Methan entsteht. Ein Teil des zumindest teilweise vergorenen Substrats wird aus dem ersten Reaktor 1 in den zweiten Reaktor 2 mittels einer Zirkulationseinrichtung 3 geleitet. Die Zirkulationseinrichtung weist dabei eine erste 4 und eine zweite Verbindungsleitung 5 auf, wobei das zumindest teilweise vergorene Substrat über die erste Verbindungsleitung 4 von dem ersten Reaktor 1 in den zweiten Reaktor 2 mit einer Pumpvorrichtung, vorzugsweise einer Schneckenexzenterpumpe, gefördert wird. Der zweite Reaktor 2 ist beheizbar und ein Inhalt des zweiten Reaktors 2 wird auf eine Temperatur von 68°C erwärmt. In dem zweiten Reaktor 2 sind hyperthermophile Bakterien, beispielsweise Clostridien, vorzugsweise Clostridium aceticum, Clostridium thermocellum und Clostridium stecorarium. Die hyperthermophilen Bakterien eignen sich, das zumindest teilweise vergorene Substrat, insbesondere die Lignocellulose, weiter aufzuschließen. Das zumindest teilweise vergorene Substrat verbleibt dazu für eine Verweilzeit von 3 Tagen im zweiten Reaktor. Dort wird die Lignocellulose von den hyperthermophilen Bakterien zumindest teilweise zu Aceton, Butanol und Essigsäure inkubiert. Das Substrat mit Essigsäure wird über die zweite Verbindungsleitung 5 mittels einer Schneckenexzenterpumpe von dem zweiten Reaktor 2 in den ersten Reaktor 1 rückgeführt. In dem ersten Reaktor 1 wird das Substrat mit Essigsäure von den mesophilen Bakterien inkubiert und es entsteht methanhaltiges Biogas mit mind. 50% Methan, 30% Kohlenstoffdioxid, 1000 ppm Schwefelwasserstoff und Spurengasen. Das Biogas wird zumindest teilweise über eine Ausleitvorrichtung 6, also eine Gasleitung, aus dem ersten Reaktor 1 isoliert und ausgeleitet. Das verbleibende zumindest teilweise vergorene Substrat wird erneut über die erste Verbindungsleitung 4 in den zweiten Reaktor 2 geleitet und dort erneut weiter aufgeschlossen, um nach einer weiteren Verweilzeit von 5 Tagen im zweiten Reaktor 2 wieder in den ersten Reaktor 1 über die zweite Verbindungsleitung 5 geleitet und dort erneut inkubiert zu werden. So wird das Substrat sowohl im ersten 1 als auch im zweiten Reaktor 2 weiter fermentiert und weiter aufgeschlossen. Diese Substratzirkulation wird derartig häufig wiederholt, bis nahezu kein oder nur wenig Biogas mehr aus dem Substrat gewonnen werden kann.

Beim Weiterleiten des zumindest teilweise vergorenen Substrats von dem ersten Reaktor 1 in den zweiten Reaktor 2 sterben die meisten mesophilen Bakterien im zweiten Reaktor 2 aufgrund der höheren Betriebstemperatur. Die hyperthermophilen Bakterien sterben zumindest größtenteils im ersten Reaktor 1, wenn diese durch die zweite Verbindungsleitung 5 in den ersten Reaktor 1 gelangen, durch die niedrigere Temperatur. Sowohl der erste Reaktor 1 als auch der zweite Reaktor 2 weist jeweils eine Rühreinrichtung 7, 8 auf, wobei die erste Rühreinrichtung 7 den Inhalt des ersten Reaktors 1 und die zweite Rühreinrichtung 8 den Inhalt des zweiten Reaktors 2 kontinuierlich durchmischen, um Ablagerungen und Verklebungen des Inhalts zu verhindern und das Substrat möglichst in einem homogenen Zustand zu halten. Im ersten Reaktor ist das Substrat in Gewichtsteilen zwanzigmal größer als das Substrat im zweiten Reaktor 2.

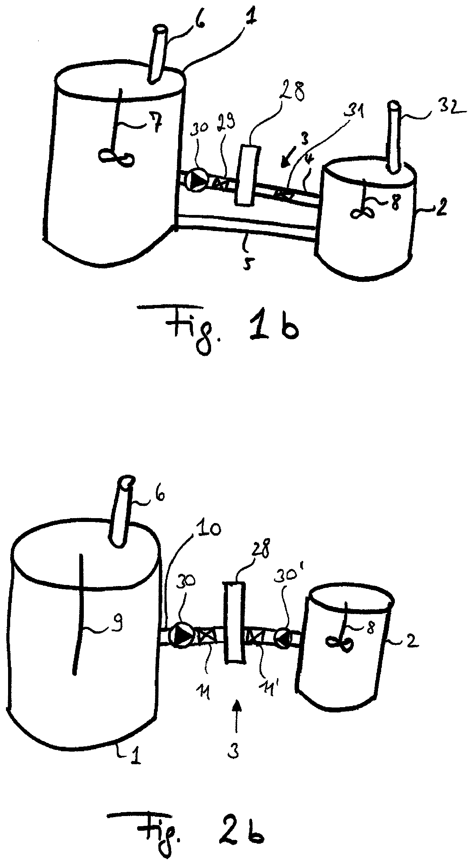

Figur 1b zeigt eine Biogasanlage, die im Wesentlichen Figur 1a entspricht, jedoch in der Verbindungsleitung 4 eine Belüftungsvorrichtung 28 umfasst. Vor der Belüftungseinrichtung 28 ist ein Ein-Wege-Ventil 29 und eine Pumpe 30 angeordnet. Hinter der Belüftungseinrichtung ist ein weiteres Ein-Wege-Ventil 30' angeordnet. Die Pumpe 30 pumpt Substrat aus dem ersten Reaktor 1 bei geöffnetem Ventil 29 und geschlossenem Ventil 31 in die Belüftungsvorrichtung. Dann wird das Ventil 29 geschlossen. Das Substrat wird in der Belüftungseinrichtung belüftet. Dabei werden die vorrangig anaeroben Bakterien aus dem ersten Reaktor 1, die sich im zu belüftenden Substrat befinden, durch die Belüftung inaktiviert oder sterben. Anschließend wird das Ventil 31 geöffnet und das belüftete Substrat in den zweiten Reaktor 2 geleitet. So kann erreicht werden, dass eine methanbildende Vergärung im zweiten Reaktor 2 weitestgehend vermieden wird. In der Rückleitung ist des Substrats von dem zweiten Reaktor 2 in den ersten Reaktor 1 ist keine Belüftung notwendig, da die Bakterien des Reaktors 2, vorrangig Clostridien, aufgrund des Temperaturunterschieds in dem zweiten Reaktor 2 und dem ersten Reaktor 1 inaktiviert werden oder sterben, da diese Bakterien höhere Temperaturen, entsprechend der Temperaturen im Reaktor 2, zum Überleben und/oder aktiven Verstoffwechseln benötigen.

Ferner weist der zweite Reaktor der Figur 1b eine Zuleitung 32 auf. Durch diese Zuleitung 32 können dem Reaktorinhalt leicht vergärbare Substanzen beigemischt werden.

Figur 2a zeigt ein weiteres Ausführungsbeispiel der Biogasanlage aus Figur 1, wobei im ersten Reaktor eine Pumpvorrichtung 9 zum Durchmischen des Substrats verwendet wird. Der Inhalt des zweiten Reaktors 2 wird mit einer Rühreinrichtung 8 durchrührt. Eine Kombination von Rühreinrichtung 8 und/oder Pumpeinrichtung 9 im ersten 1 und/oder zweiten Reaktor 2 ist somit ebenfalls denkbar für eine Biogasanlage. Dies kann insbesondere vorteilhaft sein, um entsprechend der Größe der Reaktoren eine möglichst kostengünstige und energiesparende Auswahl bezüglich einer Durchmischungsvorrichtung treffen zu können, um Herstellungskosten und Betriebskosten der Biogasanlage zu senken.

Die Figur 2b zeigt eine Biogasanlage, die im Wesentlichen der Biogasanlage der Figur 2a entspricht, jedoch eine Verbindungsleitung 10 aufweist, die zusätzlich mit einer Belüftungseinrichtung 28, zwei Pumpen 30 und zwei Zwei-Wegeventilen 11 versehen ist. Durch entsprechendes Öffnen und Schließen der Ventile 11 kann Substrat von dem ersten in den zweiten Reaktor 2 und umgekehrt gepumpt werden. Vorzugsweise wird das Substrat beim Pumpen vom ersten Reaktor 1 in den zweiten Reaktor 2 belüftet. Dafür wird das Zwei-Wege-Ventil 11 (analog zum Ein-Wege-Ventil in Figur 1b) hinter der Belüftungseinrichtung zunächst geschlossen und das Substrat von dem ersten Reaktor 1 in die Belüftungseinrichtung 28 gepumpt. Dann wird das Zwei-Wege-Ventil 11 vor der Belüftungseinrichtung 28 geschlossen und das Substrat in der Belüftungseinrichtung 28 belüftet. Nach dem Belüftungsprozess wird das Zwei-Wege-Ventil 11' hinter der Belüftungsvorrichtung geöffnet, sodass das Substrat in den zweiten Reaktor 2 geleitet wird. Um eine Zirkulation zu ermöglichen, können die Zwei-Wege-Ventile 11 und 11' zum Rückführen des Substrats vom zweiten Reaktor 2 in den ersten Reaktor 1 derart geöffnet werden, dass das Substrat nur in Richtung des ersten Reaktors 1 fließen kann. Dafür ist eine weitere Pumpe 30' vorgesehen, die das Substrat aus dem zweiten Reaktor 2 in den ersten Reaktor 1 pumpt. Damit das Substrat nicht ggf. unnötigerweise beim Rückleiten in den ersten Reaktor erneut belüftet wird, kann zudem ein weiteres Ventil an der Belüftungseinrichtung 28 vorgesehen sein, das bei einer Rückführung geschlossen ist. Ein derartiges Ventil ist in der Figur 2b nicht dargestellt, kann jedoch optional hinzugefügt werden.

Im ersten Reaktor 1 hat der Inhalt einen pH-Wert von 7. Im zweiten Reaktor 2 hat der Inhalt einen pH-Wert von 5,5. Diese pH-Werte entsprechen den bevorzugten pH-Werten der Bakterien, die im ersten bzw. zweiten Reaktor 1,2 eingebracht sind.

Der erste Reaktor 1 weist in Figur 2 ein Volumen auf, das zwanzigmal größer ist, als das Volumen des zweiten Reaktors 2. Die beiden Reaktoren 1, 2 sind in Figur 2a und 2b mit einer Zirkulationseinrichtung 3 verbunden, wobei die Zirkulationseinrichtung eine Fördereinrichtung in Form einer Schneckenexzenterpumpe aufweist und eine Verbindungsleitung 10 mit einer Zwei-Wege-Ventil-Anordnung 11. Über die Zwei-Wege-Ventil-Anordnung 11 kann eine Durchflussrichtung von dem Substrat eingestellt werden, sodass sowohl ein Substratfluss vom ersten Reaktor 1 in den zweiten Reaktor 2, als auch ein Substratfluss vom zweiten Reaktor 2 in den ersten Reaktor 1 durch die Verbindungsleitung 10 verlaufen kann. Über die Ausleitvorrichtung 6 kann Biogas, wie in Figur 1a und 1b bereits gezeigt und beschrieben, isoliert und ausgeleitet werden. Natürlich kann eine Zirkulation von Substrat auch durch ein Ausleiten von Substrat aus dem einen Reaktor, beispielsweise aus Reaktor 1, in einen Zwischenbehälter und von dem Zwischenbehälter in den anderen, beispielsweise den zweiten Reaktor 2, generiert werden. Dies kann beispielsweise dann vorteilhaft sein, wenn der erste Reaktor und der zweite Reaktor örtlich weit voneinander entfernt aufgebaut werden sollen und lange, komplexverlaufende Verbindungsleitungen nötig wären.

Zudem kann die Verweilzeit effektiv mit den Reaktorvolumina zusammenhängen. Der erste Reaktor weist ein Volumen auf, das zwanzigmal größer ist als das Volumen im zweiten Reaktor. Es kann also nur eine Teilmenge des Substrats im ersten Reaktor in den zweiten Reaktor passen, in diesem Beispiel ein zwanzigstel. Da der gesamte Inhalt des ersten Reaktors den zweiten Reaktor durchlaufen soll und je 2,5 Tage im zweiten Reaktor inkubieren soll, ergibt sich eine erste Verweilzeit im ersten Reaktor von 50 Tagen. Nach 50 Tagen wird das Substrat dann beispielsweise als Gärrest aus dem ersten Reaktor ausgeleitet.

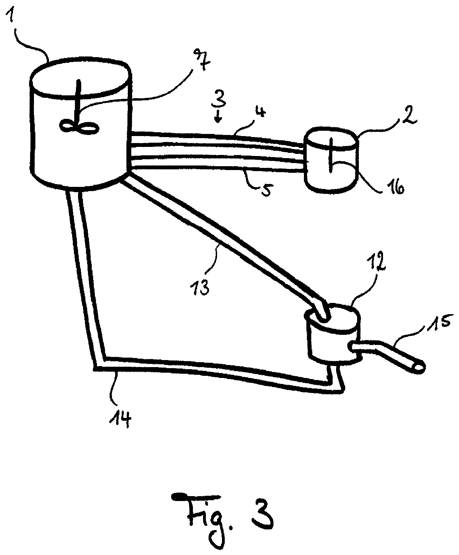

Figur 3 zeigt eine Biogasanlage mit einer Entwässerungsvorrichtung 12, die mit dem ersten Reaktor 1 über eine Ableitung 13 verbunden ist. Nach einer Vergärung im ersten Reaktor 1 und/oder zweiten Reaktor 2 entstandene Gärreste werden über die Ableitung 13 in die Entwässerungsvorrichtung 12 geleitet. Dies kann zum Beispiel zu einem Zeitpunkt geschehen, wenn eine Biogasherstellung absinkt und ein Volumenstrom in der Ausleitvorrichtung 6 einen gemessenen Grenzwert, beispielsweise bei einem Absinken von 5-10% unter den Wert des bisherigen Volumenstromes an Biogas [Nm³/h], unterschreitet. Natürlich können die Gärreste auch nach einer ersten Verweilzeit, hier beispielsweise nach 50 Tagen, aus dem ersten Reaktor in die Entwässerungsvorrichtung ausgeleitet werden. Dort wird aus den Gärresten eine Prozessflüssigkeit separiert. Diese Prozessflüssigkeit wird über eine Rückführungsvorrichtung 14 zumindest teilweise in den ersten Reaktor geleitet und dort von der Rühreinrichtung 7 mit dem Substrat gemischt. Somit kann einem Verklumpen des Substrats entgegengewirkt und ein Verhältnis eines Flüssigkeitsgehalts des Substrats zu einem Trockensubstanzgehalt des Substrats eingestellt werden. Ein fester Gärrest, also der entwässerte Gärrest, kann ferner über ein Ausleitelement 15, beispielsweise ein Rohr, aus der Entwässerungsvorrichtung 12 ausgeleitet werden. Im zweiten Reaktor 2 wird das zumindest teilweise vergorene Substrat mittels einer Pumpeinrichtung 16, vorzugsweise kontinuierlich, durchmischt.

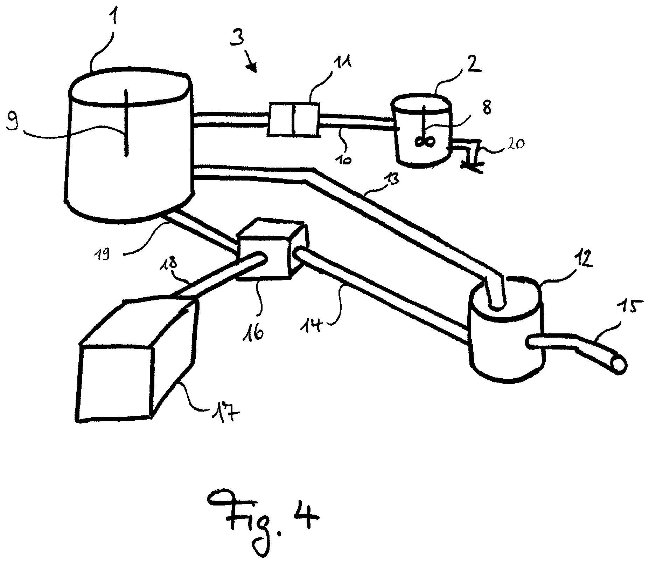

In Figur 4 ist eine Biogasanlage dargestellt, die dem Aufbau der Biogasanlage in Figur 2a entspricht, jedoch weitere Elemente, beispielsweise eine Entwässerungsvorrichtung 12, eine Mischvorrichtung 16, eine Vorbehandlungseinrichtung 17 und eine Kieselsäureausleitvorrichtung 20, umfasst. Bevor Substrat dem ersten Reaktor 1 zugeführt wird, wird es in der Vorbehandlungseinrichtung 17 auf eine Faserlänge von vorzugsweise 40 mm zerkleinert, vorzugsweise durch ein mechanisches Schneidewerk oder eine Schneidemühle. Die auf 40 mm zerkleinerten Substratfasern, insbesondere Reisstroh und Weizenstroh, werden durch ein Rohr 18 in die Mischvorrichtung geleitet. Wie auch in Figur 3 ist eine Entwässerungsvorrichtung 12 vorgesehen. Diese leitet die separierte Prozessflüssigkeit über eine Rückführungsvorrichtung 14 in die Mischvorrichtung 16. In der Mischvorrichtung 16 wird zerkleinertes Substrat und Prozessflüssigkeit gemischt, bevor es über eine Zuleitung 19 in den ersten Reaktor 1 weitergeleitet wird. Im gezeigten Beispiel wird der Mischvorrichtung 16 neben dem zerkleinerten Substrat und der Prozessflüssigkeit keine weiteren Stoffe zugeführt. Natürlich können der Mischvorrichtung 16 über weitere Leitungen verschiedene weitere Substratbestandteile, beispielsweise Gülle, zugeführt werden.

Die in Figur 4 gezeigte Biogasanlage weist ferner eine Kieselsäureausleitvorrichtung 20 auf. Diese ist vorzugsweise an den zweiten Reaktor 2 montiert, sodass Kieselsäure, die teilweise von Clostridien beim Aufschluss der Lignocellulose anfällt und methanbildungshemmend wirken kann, vor einem Weiterleiten des Substrats in den ersten Reaktor 1 ausgeschleust wird. So kann erreicht werden, dass eine Methanbildung durch die mesophilen Bakterien im ersten Reaktor 1 nicht gehemmt wird und der Wirkungsgrad der Biogasanlage kann somit verbessert werden. Die Kieselsäure kann beispielsweise durch einen Silikat- und Kieselsäurefilter separiert werden oder auch durch vorherige Fällungsreaktionen aus der Flüssigphase abgetrennt werden und im Anschluss daran aus dem zweiten Reaktor 2 ausgeleitet werden.

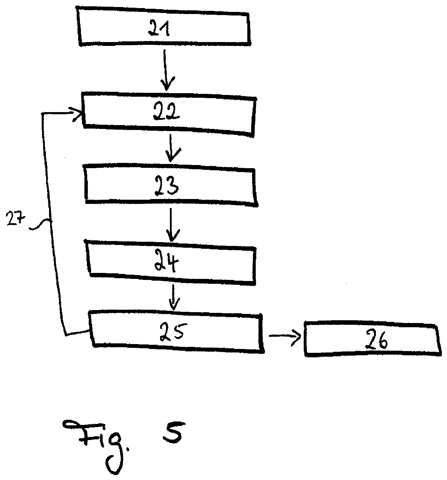

Figur 5 beschreibt schematisch einen beispielhaften Ablauf Verfahrens zum Erzeugen von Biogas mit der beschriebenen Biogasanlage. Zunächst wird in einem ersten Schritt 21 ein cellulosehaltiges Substrat, enthaltend Reisstroh und Rindergülle, für eine erste Verweilzeit von 30 Tagen bei einer Temperatur von 30 bis 48°C zumindest teilweise in einem ersten Reaktor fermentiert. In dem ersten Reaktor sind mesophile Bakterien enthalten, welche das Substrat verstoffwechseln können.

In einem zweiten Schritt 22 wird das zumindest teilweise fermentierte Substrat aus dem ersten Reaktor in einen zweiten Reaktor geleitet. Der zweite Reaktor ist beheizt und sein Inhalt hat eine Temperatur von 68°C. In dem zweiten Reaktor sind hyperthermophile Bakterien, die sich dazu eignen, das zumindest teilweise vergorenes Substrat, insbesondere die Lignocellulose, aufzuschließen.

In einem dritten Schritt 23 inkubieren die hyperthermophilen das zumindest teilweise vergorene Substrat während einer Verweilzeit von 2 Tagen des zumindest teilweise aufgeschlossene Substrats im zweiten Reaktor. Die hyperthermophilen Bakterien, insbesondere Bakterien der Gattung Clostridien schließen dabei das teilweise aufgeschlossene Substrat, insbesondere Cellulose und Lignocellulose auf und produzieren dabei neben anderen organischen Säuren, vorzugsweise Essigsäure.

In einem vierten Schritt 24 wird das zumindest teilweise aufgeschlossene Substrat aus dem zweiten Reaktor in den ersten Reaktor rückgeführt.

In einem fünften Schritt 25 wird das rückgeführte Substrat erneut im ersten Reaktor von den mesophilen Bakterien inkubiert. Dabei entsteht zumindest teilweise methanhaltiges Biogas, das in einem sechsten Schritt 26 isoliert und aus dem ersten Reaktor ausgeleitet wird.

Verbleibendes zumindest teilweise aufgeschlossenes Substrat wird wieder in den zweiten Reaktor weitergeleitet und die beschriebenen Schritte 22, 23, 24, 25 und 26 wiederholt, sodass eine Schleife 27 entsteht. Die Schritte 22 bis 26 werden solange wiederholt, bis nahezu kein methanhaltiges Biogas mehr extrahiert werden kann, beispielsweise nach einer dreimaligen Wiederholung. Danach können Gärreste ausgeleitet, die Biogasanlage erneut mit Substrat beschickt und das Verfahren erneut durchgeführt werden.

Natürlich können die Schritte - insbesondere eine Inkubation im ersten und zweiten Reaktor, also die Schritte 21, 23 und 25 - teilweise auch zeitgleich ablaufen. Zudem ist auch denkbar, dass kontinuierlich eine Zirkulation von Substrat stattfindet, Substrat inkubiert wird und Methan ausgeleitet wird. Alle Schritte 21 bis 26 können somit auch in Zeitintervallen (sog. fed batch Verfahren) ablaufen.

Folgend wird in einem Ausführungsbeispiel eine typische Substratzusammensetzung im ersten Reaktor und im zweiten Reaktor im Laufe des Verfahrens näher erläutert. Natürlich kann von den folgend gemachten Angaben in anderen Ausführungsbeispielen abgewichen werden.

Im Folgenden wird ein weiteres Ausführungsbeispiel genauer erläutert. Für die Betrachtung des Verfahrens wird ein stationärer Betrieb vorausgesetzt.
Zu Beginn des Verfahrens weist der erste Reaktor beispielsweise einen Inhalt von 3000 m³ Substrat auf. Darin befinden sich die notwendigen Mikroorganismen für den Biogasprozess. Über eine Wärmequelle werden Temperaturen von 40-48°C gewährleistet. Mittels Rührwerken und/oder Mischvorrichtungen wird das Substrat in Zeitintervallen gemischt und das gebildete Biogas aus der Flüssigphase ausgetrieben. Zudem wird eine Neuverteilung von Biomasse/Bakteriensuspension erzeugt.

Dem ersten Reaktor werden täglich 100 t Substrate zugeführt. Die Feststoffzufuhr kann dabei 14 t betragen. Dies wiederum setzt sich aus 60-65 Trockenmasse% [TM%] Reisstroh in der Mischung (mit 15% Wassergehalt), 20-25 TM% Rindergülle, 10-20 TM% Rezirkulat mit mesophilen Bakterien zusammen. Dem zweiten Reaktor sind zu Beginn des Betrachtungszeitraumes, nach einem ersten Weiterleiten ca. 100 t Substrat/Schlamm mit einem Trockenmasseanteil von 10-12 TM% zugeführt worden. Der Feststoffanteil kann sich beispielhaft aus 60-65 TM% Reisstroh, 25 TM% Gewichtsprozent Rindergülle zusammensetzen. Der verbleibende Anteil besteht aus Reststoffen aus Kurzschlussströmungen zusammen sowie aus anorganische Substanzen, Aschen sowie anderen Fremdstoffen. Abzüglich der 10t Feststoffe enthält der Zulaufstrom bis zu 90t Wasser.

Während einer Verweilzeit von beispielhaften 3 Tagen im zweiten Reaktor 2 werden bis zu 30% der Trockenmasse in lösliche Bestandteile aufgespalten. Das wird typischerweise durch die Leistungsfähigkeit der hyperthermophilen Mikroorganismen realisiert, die bei 55-80°C kultiviert werden können. Das Temperaturoptimum hängt von der Zugabe der Substrate und dessen Zusammensetzung ab. Während des Abbauprozesses können dabei bis zu 10g/l Essigsäure anfallen, welche in den ersten Reaktor 1 zurückgeführt und zu Biogas umgewandelt wird.

Die Anmeldung umfasst unter anderem folgende Aspekte:
1. Verfahren zum Erzeugen von Biogas umfassend die folgenden Schritte:
   Zumindest teilweises Vergären eines cellulosehaltigen Substrats für eine erste Verweilzeit bei einer Temperatur im Bereich von 20°C bis 40°C in einem ersten Reaktor (1) mit mesophilen Bakterien, die sich dazu eignen, aus Essigsäure Methan zu produzieren,
   Weiterleiten eines Teils des zumindest teilweise vergorenen Substrates aus dem ersten Reaktor in einen zweiten, beheizbaren Reaktor (2) mit hyperthermophilen Bakterien, wobei sich die hyperthermophilen Bakterien dazu eignen, das zumindest teilweise vergorene Substrat aufzuschließen,
   Inkubieren des zumindest teilweise vergorenen Substrates bei einer Temperatur im Bereich von 55°C bis 80°C für eine zweite Verweilzeit, wobei sich zumindest teilweise Essigsäure bildet,
   Rückführen des Substrats mit Essigsäure aus dem zweiten Reaktor (2) in den ersten Reaktor (1),
   Inkubieren des Substrats mit Essigsäure im ersten Reaktor (1),
   Isolierung von methanhaltigem Biogas aus dem ersten Reaktor (1).
2. Verfahren nach Anspruch 1, wobei das Substrat im ersten (1) und/oder zweiten Reaktor (2) mittels einer Pump- (9, 16) und/oder Rühreinrichtung (7, 8) durchmischt wird.
3. Verfahren nach Aspekt 1, wobei die Schritte
   Weiterleiten eines Teils des zumindest teilweise vergorenen Substrats aus dem ersten Reaktor (1) in einen zweiten, beheizbaren Reaktor (2) mit hyperthermophilen Bakterien, wobei sich die hyperthermophilen Bakterien dazu eignen, das zumindest teilweise vergorenen Substrat aufzuschließen, und
   Rückführen des Substrats mit Essigsäure aus dem zweiten Reaktor (2) in den ersten Reaktor (1),
   zumindest einmalig wiederholt werden.
4. Verfahren nach Aspekt 1,2 oder 3, wobei im ersten Reaktor (1) verbleibende Gärreste, die nach einer Vergärung im ersten Reaktor (1) im Wesentlichen nicht weiter durch die mesophilen Bakterien aufgeschlossen werden, aus dem ersten Reaktor (1) ausgeleitet und drainiert werden, wobei eine entsprechend separierte Prozessflüssigkeit über eine Ableitung (14) dem ersten Reaktor (1) zumindest teilweise wieder zugeführt wird.
5. Verfahren nach einem der Aspekte 1 bis 4, gekennzeichnet dadurch, dass die mesophilen Bakterien ausgewählt sind aus der Gruppe der Archaeen und vorzugsweise Methanobacteriale, Methanococcale, Methanomicrobiale, Methanocellale, Methanosarcinale, Methanoscarcina, Methanococcus, Methanobacterium Methanobrevibacter, Methanothermobacter und/oder Methanopyrale sind.
6. Verfahren nach einem der Aspekte 1 bis 5, gekennzeichnet dadurch, dass die hyperthermophilen Bakterien ausgewählt sind aus der Gruppe der Chlostridien, vorzugsweise Chlostridium aceticum, Chlostridium thermocellum und/oder Chlostridium stecorarium.
7. Verfahren nach einem der Aspekte 1 bis 6, wobei auch der Inhalt des ersten Reaktors (1) erwärmt wird.
8. Verfahren nach einem der Aspekte 1 bis 7, wobei Kieselsäure aus dem zweiten Reaktor (2) zum Herabsetzen einer Konzentration von methanbildungshemmender Kieselsäure ausgeschleust wird.
9. Verfahren nach einem der Aspekte 1 bis 8, dadurch gekennzeichnet, dass das cellulosehaltige Substrat Lignocellulose enthält.
10. Verfahren nach einem der Aspekte 1 bis 9, dadurch gekennzeichnet, dass das cellulosehaltige Substrat Stroh umfasst, insbesondere Reisstroh umfasst.
11. Verfahren nach einem der Aspekte 1 bis 10, dadurch gekennzeichnet, dass ein pH-Wert des Substrats im ersten Reaktor (1) im pH-neutralen bis leicht basischen Bereich liegt und der pH-Wert vorzugsweise mindestens 6,6 beträgt, besonders bevorzugt mindestens 6,8 beträgt und/oder maximal 8,3 beträgt, besonders bevorzugt maximal 8,0 beträgt.
12. Verfahren nach einem der Aspekte 1 bis 11, dadurch gekennzeichnet, dass ein pH-Wert des Substrats im zweiten Reaktor (2) mindestens 4,5 und/oder höchstens 6,5 beträgt.
13. Verfahren nach einem der Aspekte 1 bis 12, wobei Substratfasern vor dem Einbringen des Substrats in den ersten Reaktor (1) zerkleinert werden.
14. Biogasanlage zur Erzeugung von Biogas, enthaltend
   - einen ersten Reaktor (1) enthaltend mesophile Bakterien, die sich zur Methanherstellung aus Essigsäure, Wasserstoff und Kohlendioxid eignen,
   - einen zweiten, beheizbaren Reaktor (2) enthaltend hypothermophile Bakterien, die sich zum vorzugsweise anaeroben Vergären von einem cellulosehaltigen Substrat in Essigsäure eignen,
   - eine Zirkulationseinrichtung (3) zum Erzeugen eines Biomassekreislaufs zwischen dem ersten Reaktor (1) und dem zweiten Reaktor (2), wobei die Zirkulationseinrichtung (3) eine Fördereinrichtung und zumindest eine den ersten Reaktor (1) und den zweiten Reaktor (2) verbindende Verbindungsleitung (4, 5) umfasst und die Fördereinrichtung eingerichtet ist zum Fördern von Substrat durch die Verbindungsleitung (4, 5) sowohl von dem ersten Reaktor (1) in den zweiten Reaktor (2) als auch von dem zweiten Reaktor (2) in den ersten Reaktor (1).
15. Biogasanlage nach Aspekt 14, gekennzeichnet durch eine Entwässerungsvorrichtung (12) zum Separieren einer Prozessflüssigkeit bei einem Entwässern von Gärresten, die nach einer Vergärung im ersten Reaktor (1) im Wesentlichen nicht weiter durch die mesophilen Bakterien aufgeschlossen werden, wobei der erste Reaktor (1) zum Abführen der Gärreste mit der Entwässerungsvorrichtung (12) über eine Ableitung (13) verbunden ist.
16. Biogasanlage nach einem der Aspekte 14 oder 15, gekennzeichnet durch eine dem ersten Reaktor (1) vorgeschaltete und mit dem ersten Reaktor (1) durch eine Zuleitung (19) verbundene Mischvorrichtung (16) zum Mischen von Substrat mit einer Prozessflüssigkeit derart, dass ein Trockensubstanzgehalt des durch die Zuleitung (19) dem ersten Reaktor (1) zuführbaren Substrats einstellbar ist.
17. Biogasanlage nach Aspekt 14 oder 15, sofern dieser auf Aspekt 13 rückbezogen ist, gekennzeichnet durch eine Rückführungsvorrichtung (14) zum Rückführen der Prozessflüssigkeit in den ersten Reaktor (1).
18. Biogasanlage nach einem der Aspekte 14 bis 17, dadurch gekennzeichnet, dass die Verbindungsleitung (4, 5) eine Zwei-Wege-Ventil (11) aufweist und/oder dass die Zirkulationseinrichtung zwei Verbindungsleitungen (4, 5) zwischen dem ersten Reaktor (1) und dem zweiten Reaktor (2) umfasst.
19. Biogasanlage nach einem der Aspekte 14 bis 18, dadurch gekennzeichnet, dass die Zirkulationseinrichtung (3) eine Pumpe zum Fördern von Substrat umfasst.
20. Biogasanlage nach einem der Aspekte 14 bis 19, gekennzeichnet durch zumindest eine Rühreinrichtung (7, 8) und/oder Pumpeinrichtung (9, 16) zur Durchmischung des Substrats im ersten Reaktor (1) und/oder zweiten Reaktor (2).
21. Biogasanlage nach einem der Aspekte 14 bis 20, dadurch gekennzeichnet, dass der erste Reaktor (1) derart beheizbar ist, dass eine Temperatur von mindestens 30°C einstellbar ist
   und/oder
   dass der zweite Reaktor (2) derart beheizbar ist, dass eine Temperatur von mindestens 65°C einstellbar ist.
22. Biogasanlage nach einem der Aspekte 14 bis 21, umfassend eine Vorbehandlungseinrichtung (17) zum Zerkleinern von Substratfasern.
23. Biogasanlage nach einem der Aspekte 14 bis 22, gekennzeichnet dadurch, dass die Verbindungsleitung (4) zum Fördern von Substrat von dem ersten (1) in den zweiten Reaktor (2) eine Belüftungseinrichtung (28) umfasst.
24. Biogasanlage nach einem der Aspekte 14 bis 22, gekennzeichnet dadurch, dass der zweite Reaktor (2) eine Zuleitung (32) zum Zuleiten von leicht vergärbaren Substanzen umfasst.
25. Biogasanlage nach einem der Aspekte 14 bis 24, dadurch gekennzeichnet, dass der zweite Reaktor (2) Bakterien ausgewählt aus der Gruppe der Chlostridien, vorzugsweise aus der Gruppe Chlostridium aceticum, Chlostridium thermocellum und/oder Chlostridium stecorarium enthält.
26. Verwendung einer Biogasanlage gemäß einem der Aspekte 14 bis 25 zur Durchführung eines Verfahrens gemäß einem der Aspekte 1 bis 11.

## Patentansprüche

1. Verfahren zum Erzeugen von Biogas umfassend die folgenden Schritte:
Zumindest teilweises Vergären eines cellulosehaltigen Substrats für eine erste Verweilzeit bei einer Temperatur im Bereich von 20°C bis 55°C in einem ersten Reaktor (1) mit mesophilen Bakterien, die sich dazu eignen, aus Essigsäure Methan zu produzieren,
Weiterleiten eines Teils des zumindest teilweise vergärten Substrats aus dem ersten Reaktor in einen zweiten, beheizbaren Reaktor (2) mit hyperthermophilen Bakterien, wobei sich die hyperthermophilen Bakterien dazu eignen, das zumindest teilweise vergärte Substrat aufzuschließen,
Inkubieren des zumindest teilweise vergärten Substrats bei einer Temperatur im Bereich von 55°C bis 80°C für eine zweite Verweilzeit, wobei sich zumindest teilweise Essigsäure bildet,
Rückführen des Substrats mit Essigsäure aus dem zweiten Reaktor (2) in den ersten Reaktor (1),
Inkubieren des Substrats mit Essigsäure im ersten Reaktor (1),
Isolierung von methanhaltigem Biogas aus dem ersten Reaktor (1).

2. Verfahren nach Anspruch 1, wobei die Schritte
Weiterleiten eines Teils des zumindest teilweise vergärten Substrats aus dem ersten Reaktor (1) in einen zweiten, beheizbaren Reaktor (2) mit hyperthermophilen Bakterien, wobei sich die hyperthermophilen Bakterien dazu eignen, das zumindest teilweise vergärte Substrat aufzuschließen, und
Rückführen des Substrats mit Essigsäure aus dem zweiten Reaktor (2) in den ersten Reaktor (1),
zumindest einmalig wiederholt werden.

3. Verfahren nach Anspruch 1,2 oder 3, wobei im ersten Reaktor (1) verbleibende Gärreste, die nach einer Vergärung im ersten Reaktor (1) im Wesentlichen nicht weiter durch die mesophilen Bakterien aufgeschlossen werden, aus dem ersten Reaktor (1) ausgeleitet und drainiert werden, wobei eine entsprechend separierte Prozessflüssigkeit über eine Ableitung (14) dem ersten Reaktor (1) zumindest teilweise wieder zugeführt wird.

4. Verfahren nach einem der Ansprüche 1 bis 3, **gekennzeichnet dadurch, dass** die mesophilen Bakterien ausgewählt sind aus der Gruppe der Archaeen und vorzugsweise Methanobacteriale, Methanococcale, Methanomicrobiale, Methanocellale, Methanosarcinale, Methanoscarcina, Methanococcus, Methanobacterium Methanobrevibacter, Methanothermobacter und/oder Methanopyrale sind
und/oder
dass die hyperthermophilen Bakterien ausgewählt sind aus der Gruppe der Clostridiaceae und/oder Thermotogaceae, vorzugsweise Clostridium aceticum, Clostridium thermocellum, Clostridium und/oder Clostridium stecorarium und/oder Thermotogales.

5. Verfahren nach einem der Ansprüche 1 bis 4, **dadurch gekennzeichnet, dass** das cellulosehaltige Substrat Lignocellulose enthält
und/oder
dass das lignocellulosehaltige Substrat Stroh umfasst, insbesondere Reisstroh umfasst.

6. Verfahren nach einem der Ansprüche 1 bis 5, **dadurch gekennzeichnet, dass** ein pH-Wert des Substrats im ersten Reaktor (1) im neutralen bis leicht basischen Bereich liegt
und/oder
dass ein pH-Wert des Substrats im zweiten Reaktor (2) mindestens 4,0 und/oder höchstens 6,5 beträgt.

7. Biogasanlage zur Erzeugung von Biogas, enthaltend
- einen ersten Reaktor (1) enthaltend mesophile Bakterien, die sich zur Methanherstellung aus Essigsäure, Wasserstoff und Kohlendioxid eignen,
- einen zweiten, beheizbaren Reaktor (2) enthaltend hypothermophile Bakterien, die sich zum vorzugsweise anaeroben Vergären von cellulosehaltigen Substraten zu Essigsäure eignen,
- eine Zirkulationseinrichtung (3) zum Erzeugen eines Biomassekreislaufs zwischen dem ersten Reaktor (1) und dem zweiten Reaktor (2), wobei die Zirkulationseinrichtung (3) eine Fördereinrichtung und zumindest eine den ersten Reaktor (1) und den zweiten Reaktor (2) verbindende Verbindungsleitung (4, 5) umfasst und die Fördereinrichtung eingerichtet ist zum Fördern von Substrat durch die Verbindungsleitung (4, 5) sowohl von dem ersten Reaktor (1) in den zweiten Reaktor (2) als auch von dem zweiten Reaktor (2) in den ersten Reaktor (1).

8. Biogasanlage nach Anspruch 7, **gekennzeichnet durch** eine Entwässerungsvorrichtung (12) zum Separieren einer Prozessflüssigkeit bei einem Entwässern von Gärresten, die nach einer Vergärung im ersten Reaktor (1) im Wesentlichen nicht weiter **durch** die mesophilen Bakterien aufgeschlossen werden, wobei der erste Reaktor (1) zum Abführen der Gärreste mit der Entwässerungsvorrichtung (12) über eine Ableitung (13) verbunden ist.

9. Biogasanlage nach einem der Ansprüche 7 oder 8, **gekennzeichnet durch** eine dem ersten Reaktor (1) vorgeschaltete und mit dem ersten Reaktor (1) **durch** eine Zuleitung (19) verbundene Mischvorrichtung (16) zum Mischen von Substrat mit einer Prozessflüssigkeit derart, dass ein Trockensubstanzgehalt des **durch** die Zuleitung (19) dem ersten Reaktor (1) zuführbaren Substrats einstellbar ist.

10. Biogasanlage nach Anspruch 9 oder 8, sofern dieser auf Anspruch 8 rückbezogen ist, **gekennzeichnet durch** eine Rückführungsvorrichtung (14) zum Rückführen der Prozessflüssigkeit in den ersten Reaktor (1).

11. Biogasanlage nach einem der Ansprüche 7 bis 10, **dadurch gekennzeichnet, dass** die Verbindungsleitung (4, 5) eine Zwei-Wege-Ventil (11) aufweist und/oder dass die Zirkulationseinrichtung zwei Verbindungsleitungen (4, 5) zwischen dem ersten Reaktor (1) und dem zweiten Reaktor (2) umfasst.

12. Biogasanlage nach einem der Ansprüche 7 bis 11, **dadurch gekennzeichnet, dass** die Zirkulationseinrichtung (3) mindestens eine Pumpe zum Fördern von Substrat umfasst
und/oder
dass die Biogasanlage zumindest eine Rühreinrichtung (7, 8) und/oder Pumpeinrichtung (9, 16) zur Durchmischung des Substrats im ersten Reaktor (1) und/oder zweiten Reaktor (2) umfasst.

13. Biogasanlage nach einem der Ansprüche 7 bis 12, **dadurch gekennzeichnet, dass** der erste Reaktor (1) derart beheizbar ist, dass eine Temperatur von mindestens 30°C einstellbar ist
und/oder
dass der zweite Reaktor (2) derart beheizbar ist, dass eine Temperatur von mindestens 65°C einstellbar ist.

14. Biogasanlage nach einem der Ansprüche 7 bis 13, **gekennzeichnet dadurch, dass** die Verbindungsleitung (4) zum Fördern von Substrat von dem ersten (1) in den zweiten Reaktor (2) eine Belüftungseinrichtung (28) umfasst.

15. Verwendung einer Biogasanlage gemäß einem der Ansprüche 7 bis 14 zur Durchführung eines Verfahrens gemäß einem der Ansprüche 1 bis 11.
